# EUROPÄISCHE PATENTSCHRIFT

(11) **EP 3 033 022 B1**
(45) Veröffentlichungstag und Bekanntmachung des Hinweises auf die Patenterteilung: **02.05.2018**
(21) Anmeldenummer: 15790100.0
(22) Anmeldetag: 02.11.2015
(51) Int. Cl.: A61B 18/14, A61B 17/28

(54) **BIPOLARES CHIRURGISCHES INSTRUMENT MIT MEHRWEGHANDGRIFF UND EINWEGWERKZEUG**
BIPOLAR SURGICAL INSTRUMENT COMPRISING A REUSABLE HANDLE AND A SINGLE-USE TOOL
INSTRUMENT CHIRURGICAL BIPOLAIRE COMPORTANT UNE POIGNÉE À USAGES MULTIPLES ET OUTIL À USAGE UNIQUE

(30) Priorität: 04.11.2014 DE 102014116065
(43) Veröffentlichungstag der Anmeldung: 22.06.2016
(73) Patentinhaber: Aesculap AG, 78532 Tuttlingen (DE)
(72) Erfinder: SCHWARZ, Tina, 78628 Rottweil (DE)
(74) Vertreter: Winter, Brandl, Fürniss, Hübner, Röss, Kaiser, Polte - Partnerschaft mbB
(86) Internationale Anmeldenummer: PCT/EP2015/075411
(87) Internationale Veröffentlichungsnummer: WO 2016/071263

(56) Entgegenhaltungen:
- EP-A1- 0 697 199
- EP-A2- 2 642 418
- DE-U1-202009 014 091
- US-A- 6 074 386
- US-B1- 6 238 394

## Beschreibung

Die Erfindung betrifft ein bipolares chirurgisches Instrument mit einem mehrfach verwendbaren Handgriff und einem einmal verwendbaren Werkzeug sowie einer Verbindungsvorrichtung dazwischen, und bezieht sich insbesondere auf ein bipolares chirurgisches Instrument mit einer Verbindungsvorrichtung, mittels welcher ein zum einmaligen Gebrauch vorgesehenes chirurgisches Schaftwerkzeug (mit eigenem Schaftabschnitt) steckbar mit einem wiederverwendbaren Handstück/Handgriff verbunden werden kann. Des Weiteren betrifft die Erfindung einen wieder verwendbaren Handgriff sowie ein Schaftwerkzeug eines solchen chirurgischen Instruments.
Im Bereich bipolarer chirurgischer Instrumente insbesondere der minimalinvasiven Bauart sind aus dem Stand der Technik beispielsweise Instrumente mit bipolaren Rohrschaftpinzetten/-zangen als Rohrschaftwerkzeuge bekannt, welche über einen Betätigungselemente aufweisenden Handgriff am proximalen Ende eines Rohrschafts bedien- und/oder betätigbar sind. Hierbei befindet sich am distalen Ende des Rohrschafts eine mehrteilige, etwa scheren-, zangen- oder pinzettenförmige Anordnung von gegeneinander beweglich am Rohrschaft angelenkten Elementen/Branchen. Im Innern des Rohrschafts verläuft ein Getriebe in Form einer Zug-/Druckstange, die die beweglich am Rohrschaft angelenkten Elemente/Branchen mit entsprechenden Betätigungselementen (Hebel, Tasten, Scherengriffe, etc) am Handgriff koppelt. Eine Manipulation der Betätigungselemente am Handgriff führt sodann zu einer entsprechend umgesetzten Bewegung der beweglich angelenkten Elemente/Branchen am Anwendungsort, etwa einer Schneide-, Greif- und/oder Drehbewegung am oder im Gewebe eines Patienten.
Die Elemente/Branchen sind ferner mit Elektroden bestückt, die über elektrische Leiter innerhalb des Werkzeugschafts mit einer Steuerungselektrik im Handgriff gekoppelt sind, um wahlweise mit einem elektrischen Strom (HF-Strom) beaufschlagt werden zu können. Bei solchen bekannten Anordnungen bipolarer Rohrschaftinstrumente kommen bislang lediglich feste Verbindungen und/oder Kontaktierungen zwischen dem Handgriff und dem Rohrschaftwerkzeug durch beispielsweise Verschweißen und/oder Verlöten der jeweiligen (stromführenden) Bauteile zum Einsatz.

Aus der HF-Chirurgie sind ferner Federelemente für monopolare Instrumenten-Stecksysteme bekannt, mittels welchen ein Schaft eines Rohrschaftwerkzeugs mit einem Handgriff verbindbar ist. Steckverbindungen für monopolare Elektroden sind entsprechend verschiedenen Elektrodendurchmessern und -formen verfügbar. Die monopolaren Elektroden können dort ohne weitere bewegliche Teile in eine federkorbartige Halteanordnung innerhalb der Steckverbindung gesteckt und auch wieder aus dieser federkorbartigen Anordnung gezogen werden.

Aus der Druckschrift US 6 238 394 B1 ist ein elektrochirurgisches Instrument bekannt mit einem Universalhandgriff, der zur Anbringung und Entfernung verschiedener Rohrschaftwerkzeuge für die Ausführung unterschiedlicher elektrochirurgischer Prozeduren ausgelegt ist. Für das Anbringen und Entfernen ist der Handgriff mehrteilig ausgebildet, mit oberen konkaven Abschnitten, die zum Aufnehmen und Halten der Rohrschaftwerkzeuge ausgestaltet sind. Die Werkzeuge sind mit ähnlichen äußeren Gehäuseformen versehen, so dass sie alle in die konkaven Abschnitte hineinpassen. Die Handgriffteile werden von schnappbaren Rückhaltemechanismen zusammengehalten und können in gleicher Weise wieder voneinander gelöst werden. Der Handgriff kann eine Vielzahl von Kernwerkzeugen aufnehmen, die Teile einer Familie von Werkzeugen für sowohl monopolare als auch bipolare elektrochirurgische Prozeduren umfassen können.

Außerdem ist aus der Druckschrift EP 0 697 199 B1 ein chirurgisches Bipolarinstrument bekannt mit einem rohr-/schaftförmigen Gehäuse, in das ein mit zwei Elektroden versehener Elektrodenkopf unter Ausbildung einer elektrischen Verbindung zwischen den Elektroden und zwei im Gehäuse angeordneten Leitern lösbar einsteckbar ist. Dazu wird ein Leiter durch einen leitenden, außenseitig elektrisch isolierten Rohrschaft und ein weiterer Leiter durch einen im Rohrschaft elektrisch isoliert von diesem gelagerten Stab gebildet. Eine Elektrode weist bei vollständig eingestecktem Elektrodenkopf an der Innenseite des Rohrschafts elektrisch leitend anliegende, federnd in radialer Richtung bewegbare, mit einem Vorsprung in einen Rücksprung der Innenwand des Rohrschafts oder des Stabs eingreifende und die Elektrode dadurch gegen eine axiale Verschiebung im rohrförmigen Gehäuse festlegende Einsteckelemente auf. Der Rohrschaft und der Stab sind zwischen einer Verriegelungsposition und einer Freigabeposition in Instrumenten-Längsrichtung relativ zueinander verschiebbar, wobei der Rohrschaft und der Stab in der Verriegelungsposition die Einsteckelemente mit dem Vorsprung in den Rücksprung eingreifend radial unverschiebbar zwischen sich einschließen, während sie in der Freigabeposition ein radiales Austreten des Vorsprungs aus dem Rücksprung zulassen. Der Stab liegt in der Verriegelungsposition an der anderen Elektrode elektrisch leitend an.

Schließlich ist aus der US 6,074,386 ein elektrochirurgisches Instrument dieser Gattung bekannt, welches den zum vorliegenden Erfindungsgegenstand nächstkommenden Stand der Technik darstellt.

Während eine elektrische Kontaktierung einer wechselbaren monopolaren und/oder bipolaren Elektrode demnach bereits verfügbar ist, besteht bei bipolaren chirurgischen Instrumenten, insbesondere bestrombaren, betätigbaren und/oder für eine Drehbewegung ausgelegten bipolaren Rohrschaftinstrumenten, nach wie vor Bedarf an einem Stecksystem zur Verbindung bzw. Trennung eines zur einmaligen Verwendung vorgesehenen Rohrschaftwerkzeugs mit bzw. von einem wiederverwendbaren Handgriff, das sowohl eine elektrische Kontaktierung, eine Fixierung (mechanische Kopplung) des Werkzeugs am Handgriff und zeitgleich eine Dreh- Schwenk- und/oder Verschiebbarkeit von Teilen des Werkzeugs erlaubt.

Der Erfindung liegt daher die Aufgabe zu Grunde, ein chirurgisches bipolares Instrument sowie ein Schaftwerkzeug für ein solches bipolares chirurgisches Rohrschaftinstrument zu schaffen, wodurch ein zur einmaligen Verwendung vorgesehenes Schaftwerkzeug mit Schaftabschnitt lösbar mit einem wiederverwendbaren Handstück verbindbar ist, und welches gleichzeitig eine Fixierung/mechanische Kopplung des Schaftabschnitts, eine elektrische Kontaktierung bipolar zu bestromender Elemente/Elektroden und wenigstens einen rotatorischen Freiheitsgrad für den Schaftabschnitt innerhalb der Verbindungsvorrichtung bereitstellt.

Diese Aufgabe wird erfindungsgemäß durch die in den Patentansprüchen 1 und 10 angegebenen Merkmale gelöst. Vorteilhafte Weiterbildungen der Erfindung sind Gegenstand der Unteransprüche.

Der Erfindung liegt die allgemeine Idee zugrunde, unter Verwendung zweiter Federelemente/Federkörbe/Federkragen/Federmanschetten oder auch Federlamellen zur Fixierung/mechanischen Kopplung eines Rohrschaftwerkzeugs in einem Handgriff und zur elektrischen Bestromung von Elektroden am Rohrschaftwerkzeug sowie vorzugsweise mit Freiheitsgraden wenigstens zum Rotieren des Rohrschaftwerkzeugs im Instrumentenhandgriff um dessen Schaftlängsachse und ggf. zum Bewegen von Werkzeugbranchen (an denen die Elektroden angeordnet sind) ein Stecksystem zu schaffen, welches vor einem chirurgischen Eingriff verbunden wird und nach dem Eingriff wieder getrennt werden kann. Wenigstens ein Federelement/-korb/-kragen/-manschette/-lamelle (nachfolgend der Einfachheit halber nur noch Federkorb bezeichnet) ermöglicht bzw. vorzugsweise die zwei Federkörbe ermöglichen dabei die Fixierung (in Schaftlängsrichtung)/mechanische Kopplung eines Werkzeugschafts in einem Handstück, eine feste mechanische Kontaktierung von unbeweglichen Teilen und eine Schleifkontaktierung wenigstens an beweglichen Teilen des Schafts, um Strom zu leiten.

Das bipolare Rohrschaftwerkzeug, beispielsweise eine bipolare Rohrschaftpinzette oder Fasszange, zur einmaligen Anwendung bzw. zum Einmalgebrauch durch das Stecksystem, mithin einer Verbindungsvorrichtung oder einer Kopplungsvorrichtung, ist damit lösbar so mit einem wiederverwendbaren Handstück oder Handgriff verbindbar, dass das Schaftwerkzeug nach der Anwendung entsorgt und das Handstück wiederaufbereitet und sodann wiederverwendet werden kann. Das Stecksystem ist so ausgelegt, dass ein jeweils neues Schaftwerkzeug in dem Handstück befestigt werden kann, d. h. dort fixiert/mechanisch gekoppelt und gehalten wird, dass eine elektrische Kontaktierung zu bestromender Teile des Schaftabschnitts über wenigstens ein Federelement innerhalb des Stecksystems realisiert wird, und gleichzeitig über einen Schleifkontakt eine Drehbarkeit von um die Längsachse des Schaftabschnitts drehbaren Elementen des Instruments unter Aufrechterhaltung deren Bestromung möglich ist.

In anderen Worten ausgedrückt wird erfindungsgemäß u. a. ein manueller Handgriff eines bipolaren elektrochirurgischen Instruments vorzugsweise der Minimalinvasivbauart vorgeschlagen mit einer Strombeaufschlagungsvorrichtung für das wahlweise Beaufschlagen eines chirurgischen Werkzeugs mit einem elektrischen Strom (Gleich- oder HF-Strom) mittels eines vorzugsweise am Handgriff angeordneten Stromschalters und einem Aufnahmefutter, in welches das chirurgische Werkzeug an dessen Werkzeugschaft mechanisch ein- und aussteckbar und dabei elektrisch an der Strombeaufschlagungsvorrichtung ein- und auskoppelbar ist. Das Aufnahmefutter hat zwei in Einsteckrichtung des Werkzeugschafts axialbeabstandete, radial elastisch nachgebende sowie elektrisch voneinander isolierte Aufnahme-/Lagerungselemente zur radialen und/oder axialen Führung und Sicherung des Werkzeugschafts, welche mit der Strombeaufschlagungsvorrichtung elektrisch verbunden sind, um einen elektrischen Strom auf das eingesteckte Werkzeug zu übertragen.

Dadurch kann der Werkzeugschaft einfach in das Aufnahmefutter eingesteckt und federelastsich gegen ein unbeabsichtigtes Herausfallen gesichert werden. Gleichzeitig schaffen die elastisch nachgebenden (radial aufweitbaren) Aufnahme-/Lagerungselemente den elektrischen Anschluss für das Werkzeug, sodass ein Werkzeugwechsel einfach und sicher ausführbar ist.

Vorzugsweise wird also ein Handgriff eines bipolaren chirurgischen Instruments vorzugsweise der Minimalinvasivbauart vorgeschlagen mit einer Strombeaufschlagungsvorrichtung für das wahlweise Beaufschlagen eines chirurgischen Werkzeugs mit einem elektrischen Strom mittels eines vorzugsweise am Handgriff angeordneten Stromschalters und einem Aufnahmefutter, in welches das chirurgische Werkzeug an dessen Werkzeugschaft mechanisch ein- und aussteckbar und dabei elektrisch an der Strombeaufschlagungsvorrichtung ein- und auskoppelbar ist, wobei das Aufnahmefutter zwei in Einsteckrichtung des Werkzeugschafts axial beabstandete, radial elastisch nachgebende sowie elektrisch voneinander isolierte Aufnahme-/Lagerungselemente zur radialen und gleichzeitig axialen Führung und Sicherung des Werkzeugschafts hat, die dazu angeordnet sind, eine Reibschluss erzeugende Einspannkraft zu erzeugen und welche des Weitern gleichzeitig mit der Strombeaufschlagungsvorrichtung (118, 156) elektrisch und bipolar verbunden und dazu angeordnet sind, einen elektrischen Strom auf das eingesteckte Werkzeug zu übertragen.

Vorteilhaft ist es, wenn die Aufnahme-/Lagerungselemente jeweils eine radial elastisch aufweitbare Schaftmanschette vorzugsweise in Form eines Schleifrings, Kugellagers mit radial elastisch verschiebbaren Kugeln oder eines Federkorbs ausbilden. Vorzugsweise können die beiden Aufnahme-/Lagerungselemente dafür angepasst sein, zueinander unterschiedlich große Radialkräfte auf den eingesteckten Werkzeugschaft auszuüben. Dadurch ist es möglich, beispielsweise einen Werkzeugschaft bestehend aus zwei ineinander gesteckten Schaftstäben an seinem einen Stab stärker (fest) und an seinem anderen Stab schwächer (lose) zu greifen, um z. B. eine Axialverschiebung des schwächer ergriffenen Stabs relativ zum stärker ergriffenen Stab am Handgriff zu ermöglichen.

Vorteilhaft ist es, wenn wenigstens eines der Aufnahme-/Lagerungselemente einen radial einwärts gerichteten Vorsprung oder eine radial einwärts gerichtete Ausbauchung hat. Vorzugsweise kann es hierbei vorgesehen sein, dass der Vorsprung/die Ausbauchung dafür angepasst ist, mit einer radialen Einkerbung/Hinterschneidung in Rasteingriff für ein axiales Sichern des Werkzeugschafts im Aufnahmefutter des Handgriffs zu kommen. Auf diese Weise wird die axiale Sicherung des nicht ausschließlich durch Reibschluss sondern durch einen federelastisch bewirkten Formschluss erreicht.

Des Weiteren wird erfindungsgemäß ein Werkzeug eines bipolaren chirurgischen Instruments vorzugsweise der Minimalinvasivbauart vorgeschlagen, das dafür angepasst ist, in den Handgriff des chirurgischen Instruments gemäß vorstehender Definition eingesteckt zu werden. Das Werkzeug hat einen Elektroden besetzten oder aufweisenden Werkzeugkopf am distalen Ende des Werkzeugschafts, dessen proximales Schaftende in das Aufnahmefutter des Handgriffs einsteckbar ist. Erfindungsgemäß hat der Werkzeugschaft u. a. zwei axial beabstandete, elektrisch voneinander isolierte Schaftabschnitte, die dafür angepasst sind, mit den beiden Aufnahme-/Lagerungselementen im Aufnahmefutter des Handgriffs in mechanischen Führungs- und/oder Sicherungseinriff und gleichzeitig in elektrischen Kontakt zu kommen, um die Elektroden am Werkzeugkopf mit der Strombeaufschlagungsvorrichtung zu verbinden. Vorzugsweise ist das Werkzeug als Ein-Weg-Artikel konzipiert.

Weiter vorzugsweise wird demnach ein Werkzeug eines bipolaren chirurgischen Instruments vorzugsweise der Minimalinvasivbauart vorgeschlagen, das dafür angepasst ist, in einen Handgriff des chirurgischen Instruments vorzugsweise gemäß einem der vorstehend beschriebenen Ausführungsformen eingesteckt zu werden, mit einem Elektroden besetzten oder aufweisenden Werkzeugkopf am distalen Ende eines Werkzeugschafts, dessen proximales Schaftende in das Aufnahmefutter des Handgriffs einsteckbar ist, wobei der Werkzeugschaft zwei axial beabstandete, elektrisch voneinander isolierte Schaftabschnitte hat, die dazu angepasst sind, mit den beiden Aufnahme-/Lagerungselementen vorzugsweise gemäß einem der vorstehend beschriebenen Ausführungsformen im Aufnahmefutter des Handgriffs in eine auf den Werkzeugschaft wirkende Einspannkraft zu erzeugen den Werkzeugschaft radial und gleichzeitig axial auf der Grundlage eines durch die Einspannkraft erzeugten Reibschlusses zu führen und/oder zu sichern und weiter gleichzeitig elektrisch und bipolar zu kontaktieren, um die Elektroden am Werkzeugkopf mit der Strombeaufschlagungsvorrichtung zu verbinden.

Schließlich wird erfindungsgemäß ein elektrochirurgisches Instrument der bipolaren Bauart vorgeschlagen mit einem Handgriff und einen Werkzeug gemäß der vorstehenden Definitionen. Vorteilhaft wäre es dabei, wenn der Handgriff als mehrfach und das Werkzeug als nur einmal verwendbares Produkt vorgesehen ist.

In anderen Worten wird das bipolare Rohrschaftinstrument in dem Handgriff über zwei (federelastische) Kontakt-(lamellen)-einrichtungen mechanisch gehalten/gelagert und gleichzeitig bestromt. Eine Kontaktlamelleneinrichtung mit einer vorzugsweise geringen Kraft ist an einer Zug-/Druckstange lokalisiert. Die andere Kontaktlamelleneinrichtung mit einer vorzugsweise großen Kraft ist am Außenrohr des Rohrschafts lokalisiert, in welchem die Zug-/Druckstange gelagert ist.

Die beiden Teile (Außenrohr und Zug-/Druckstange) sind gegeneinander elektrisch isoliert. Die Kraft der anderen Kontaktlamelleneinrichtung ist groß genug, um den gesamten Schaft während der Anwendung im Handgriff/-stück zu fixieren oder lediglich rotieren zu lassen. Sie ist außerdem klein genug, um den Schaft wieder aus dem Handstück (manuell) lösen zu können, wenn dies beabsichtigt ist. Die Zug-/Druckstange wird über einen Schleifkontakt der einen Kontaktlamelleneinrichtung bestromt, in welchem er axial verschiebbar ist.

In einer Modifikation wird die Bestromung nicht über die Stangen/Schäfte selbst sondern mittels elektrischer Leitern, beispielsweise Litzen, und Buchsen zur Kontaktierung derart realisiert, dass nicht die Zug-/Druckstange und das Außenrohr, sondern die an die Buchsen angeschlossenen elektrischen Leiter im Rohr den Strom leiten. In diesem Fall sind die Buchsen vorzugsweise sämtlich am Außenrohr angeordnet, das fest/nur rotierend im Handgriff steckt und daher nicht axial verschoben werden kann. Dadurch kann der Schleifkontakt (vorher an der Zug-/Druckstange) in diesem Ausführungsbeispiel entfallen.

Vorteile ergeben sich insbesondere dadurch, dass Schaft und Handgriff leicht voneinander trennbar sind, und dass über ein Lagerungselement mehrere Funktionen (Halten, Lagern, elektrisch Kontaktieren) führbar bzw. realisierbar sind. Erfindungsgemäß wird somit ein bipolares chirurgisches Instrument vorgeschlagen mit einem Handgriff vorzugsweise mit einem festen Griffstück und einem beweglichen Betätigungshebel zur Betätigung einer ersten und/oder einer zweiten Branche an dem bezüglich des Handgriffs/Handstücks distalen Ende des bipolaren chirurgischen Werkzeugs; eine schachtförmige Führungseinrichtung (Aufnahmefutter), in der innenseitig eine Lagerungseinrichtung zur axialverschiebbaren und/oder drehbaren Aufnahme des Werkzeugschafts angeordnet ist, eine Kraftübertragungseinrichtung, die proximalseitig am festen Griffstück und/oder dem beweglichen Betätigungshebel des Handgriffs und distalseitig an der ersten und/oder der zweiten Branche angelenkt ist; wobei die Führungseinrichtung (oder auch Verbindungsvorrichtung) dafür angepasst ist, dass der Werkzeugschaft einschließlich der darin untergebrachten Kraftübertragungseinrichtung in die Lagerungseinrichtung steckbar und lösbar ist, wobei diese steckbare und lösbare Kopplung im gekoppelten Zustand eine Haltekraft zum Halten des Werkzeugschafts (und ggf. der Kraftübertragungseinrichtung in der Verbindungsvorrichtung, eine bipolare elektrische Kontaktierung distalseitig des Werkzeugschafts zu bestromender Teile des chirurgischen Instruments und Freiheitsgrade einer Längsbewegung und/oder einer rotatorischen Bewegung des (rohrförmigen) Werkzeugschafts und/oder der Kraftübertragungseinrichtung bereitstellt. Vorteilhaft wird somit ein Stecksystem zum Verbinden und Trennen eines bestromten, zur einmaligen Verwendung vorgesehenen Instrumentenwerkzeugs und eines wiederverwendbaren Handgriffs durch die Kontaktlamellen verwirklicht, wobei die Verbindung gleichzeitig eine Drehbewegung von zumindest Teilen des Werkzeugs, die Bestromung des Werkzeugs über
- wenigstens einen (quasi) festen Kontakt und
- einen (axial wirkenden) Schleifkontakt oder, alternativ hierzu einen weiteren (quasi) festen Kontakt, und
- die Fixierung des Werkzeugs im Handgriff
erlaubt.

Bevorzugt ist der rohrförmige Werkzeugschaft durch ein Isolationselement elektrisch von der darin gelagerten Kraftübertragungseinrichtung isoliert; weist die Verbindungsvorrichtung eine erste Kontaktlamelleneinrichtung und eine zweite Kontaktlamelleneinrichtung mit jeweils einer Vielzahl einzelner, federnd gehaltener Kontaktlamellen auf; ist die erste Kontaktlamelleneinrichtung an einer ersten Position in der Verbindungsvorrichtung konzentrisch zu der Längsachse des rohrförmigen Werkzeugschafts angeordnet und kontaktiert an dieser Position den Außenumfang des rohrförmigen Werkzeugschafts; und ist die zweite Kontaktlamelleneinrichtung an einer zweiten Position in der Verbindungsvorrichtung konzentrisch zu der Längsachse der Kraftübertragungseinrichtung angeordnet und kontaktiert an dieser Position den Außenumfang der Kraftübertragungseinrichtung. Vorteilhaft wird dadurch eine Steckverbindung realisiert, die eine bipolare elektrische Versorgung des Instruments über nur zwei Positionen in der Verbindungseinrichtung herstellt, wobei an diesen Positionen gleichzeitig weitere Funktionalität bereitgestellt wird. Vorteilhaft kann es dabei beispielsweise sein, wenn der rohrförmige Werkzeugschaft zumindest teilweise elektrisch leitend ausgebildet ist. Auf diese Weise kann mittels des Werkzeugschafts besonders einfach ein elektrischer Strom auf mindestens ein Werkzeugelement (Elektrode am Werkzeugkopf/Branche) des chirurgischen Instruments übertragen werden. Insbesondere kann vorgesehen sein, dass der Werkzeugschaft als eine erste Stromleitung und die darin gelagerte Kraftübertragungseinrichtung als eine zweite Stromleitung zur Versorgung der mindestens zwei Werkzeugbranchen des chirurgischen Instruments mit Strom ausgebildet sind. Der Werkzeugschaft und die Kraftübertragungseinrichtung sind vorzugsweise elektrisch voneinander isoliert.

Weiter bevorzugt weist die erste Kontaktlamelleneinrichtung in einem ungekoppelten Zustand einen Innendurchmesser auf, der kleiner ist als der Außendurchmesser des rohrförmigen Werkzeugschafts, und erzeugen im gekoppelten Zustand die Vielzahl der federnd gehaltenen Kontaktlamellen eine auf den Außenumfang des rohrförmigen Werkzeugschafts wirkende Halte-/Einspannkraft, die so bemessen ist, dass der rohrförmige Werkzeugschafs einerseits für die Dauer einer Anwendung des chirurgischen Instruments sicher in der Verbindungsvorrichtung (Aufnahmefutter) gehalten und bestromt wird, und andererseits der rohrförmige Werkzeugschaft nach der Anwendung aus dem gekoppelten Zustand lösbar bleibt. Vorteilhaft genügt somit nur eine entsprechend stark ausgelegte Kontaktlamelleneinrichtung, um den rohrförmigen Werkzeugschaft fest in der Verbindungsvorrichtung zu halten, so dass die andere Kontaktlamellenvorrichtung entsprechend schwächer und damit preiswerter und/oder funktionsoptimiert dimensioniert werden kann.

Erfindungsgemäß ist am Außenumfang des rohrförmigen Werkzeugschafts eine Vertiefung / eine axial wirkende Hinterschneidung bildende Ausformung vorgesehen, in welche bevorzugt die Vielzahl der federnd gehaltenen Kontaktlamellen im gekoppelten Zustand rastbar ist. Eine Rastung gibt dem Anwender vorteilhaft eine taktile Rückmeldung über den betriebsfähig hergestellten Verbindungs- oder Kopplungszustand (in axialer Richtung). Es kann somit vor einer Anwendung vermieden werden, dass das Werkzeug versehentlich nicht vollständig mit dem Handgriff mechanisch verbunden und/oder nicht wie vorgesehen bestromt ist. Die Gefahr von Anwendungsfehlern am Patienten wird dadurch verringert.

Weiter bevorzugt weist die zweite Kontaktlamelleneinrichtung in einem ungekoppelten Zustand einen Innendurchmesser auf, der kleiner ist als der Außendurchmesser der Kraftübertragungseinrichtung, und erzeugen im gekoppelten Zustand die Vielzahl der federnd gehaltenen Kontaktlamellen eine auf den Außenumfang der Kraftübertragungseinrichtung wirkende Einspannkraft, die so bemessen ist, dass die Vielzahl der federnd gehaltenen Kontaktlamellen einerseits eine bestrombare Schleifkontaktierung mir der Kraftübertragungseinrichtung ausbilden und andererseits die Kraftübertragungseinrichtung schleifkontaktiert dreh-/ axialverschiebbar bleibt. Die Auslegung der zweiten Kontaktlamelleneinrichtung als lediglichen Schleifkontakt vereinfacht vorteilhaft den konstruktiven Aufbau der Verbindungsvorrichtung.

Alternativ bevorzugt kann es sein, dass außenseitig des rohrförmigen Werkzeugschafts an einer ersten Position eine erste Kontaktbuchseneinrichtung angeordnet ist und an einer zweiten Position eine zweite Kontaktbuchseneinrichtung angeordnet ist, wobei die erste Kontaktbuchseneinrichtung und die zweite Kontaktbuchseneinrichtung durch ein sich über zumindest eine vorbestimmte Länge auf dem Außenumfang des rohrförmigen Werkzeugschafts erstreckendes Isolationselement elektrisch vom rohrförmigen Werkzeugschafts isoliert sind; ist die erste Kontaktbuchseneinrichtung mittels einer ersten Leiterverbindung elektrisch mit einer der Branchen verbunden und ist die zweite Kontaktbuchseneinrichtung mittels einer zweiten Leiterverbindung mit der anderen der Branchen verbunden; weist die Verbindungsvorrichtung eine erste Kontaktlamelleneinrichtung und eine zweite Kontaktlamelleneinrichtung mit jeweils einer Vielzahl einzelner, federnd gehaltener Kontaktlamellen auf; ist die erste Kontaktlamelleneinrichtung an der ersten Position in der Verbindungsvorrichtung konzentrisch zu der Längsachse des rohrförmigen Werkzeugschafts angeordnet und kontaktiert an dieser Position den Außenumfang der ersten Kontaktbuchseneinrichtung; und ist die zweite Kontaktlamelleneinrichtung an der zweiten Position in der Verbindungsvorrichtung konzentrisch zu der Längsachse des rohrförmigen Werkzeugschafts angeordnet und kontaktiert an dieser Position den Außenumfang der zweiten Kontaktbuchseneinrichtung. Zwei Stromführungen für die wenigstens zwei Elektroden/Branchen und eine gegenseitige Isolation werden vorzugsweise durch ein einziges Element, nämlich den rohrförmigen Werkzeugschaft, ersetzt. Daher kann die Kraftübertragungseinrichtung massiver ausgelegt werden, so dass eine höhere Stabilität des chirurgischen Instruments in der Anwendung und somit eine geringere elastische Verformung und eine bessere Taktilität erreicht werden.

Ferner bevorzugt weist in einem ungekoppelten Zustand die erste Kontaktlamelleneinrichtung einen Innendurchmesser auf, der kleiner ist als der Außendurchmesser der ersten Kontaktbuchseneinrichtung, und weist die zweite Kontaktlamelleneinrichtung einen Innendurchmesser auf, der kleiner ist als der Außendurchmesser der zweiten Kontaktbuchseneinrichtung; und erzeugen im gekoppelten Zustand die Vielzahl der federnd gehaltenen Kontaktlamellen der ersten und der zweiten Kontaktlamelleneinrichtung jeweils eine auf den Außenumfang der ersten und der zweiten Kontaktbuchseneinrichtung wirkende Haltekraft, die so bemessen ist, dass der rohrförmige Werkzeugschaft einerseits für die Dauer einer Anwendung des chirurgischen Instruments sicher in der Verbindungsvorrichtung axial gehalten und bestromt wird und andererseits der rohrförmige Werkzeugschaft ggf. rotierbar sowie nach der Anwendung aus dem gekoppelten Zustand lösbar bleibt. Die Aufteilung der Haltekraft auf zwei Kontaktlamelleneinrichtungen ist dahingehend vorteilhaft, dass bei einer Schwächung einer Kontaktlamelleneinrichtung noch immer eine Haltekraft durch die zweite Kontaktlamelleneinrichtung bereitgestellt wird. Zum Einen werden somit die einzelnen Kontaktlamelleneinrichtungen gleichmäßiger und/oder weniger stark belastet, und wird gewährleistet, dass bei gegebenenfalls einem Bruch einer der Kontaktlamelleneinrichtungen während einer Anwendung das Instrument durch die andere Kontaktlamelleneinrichtung weiter axial gehalten wird und somit während der Anwendung am Patienten nicht unkontrolliert herausfallen kann.

Vorteilhaft sind die erste Kontaktlamelleneinrichtung und die zweite Kontaktlamelleneinrichtung gleich dimensioniert und erzeugen jeweils dieselbe Haltekraft (Einspannkraft auf den Werkzeugschaft). Hierdurch kann vorteilhaft im Reparatur- oder Wartungsfall nur eine Teilenummer vorgesehen werden, und vereinfacht sich die konstruktive Auslegung der Verbindungsvorrichtung.

Alternativ können die erste Kontaktlamelleneinrichtung und die zweite Kontaktlamelleneinrichtung unterschiedlich dimensioniert sein und jeweils unterschiedliche Haltekräfte erzeugen. Diese Möglichkeit kann eine einfachere Anpassung der Verbindungsvorrichtung an verschiedene Werkzeuge erlauben, und beispielsweise in Form modulartig wechselbarer Verbindungsvorrichtungen Kompatibilität und Zugang zu Werkzeugen von Fremdherstellern eröffnen.

Erfindungsgemäß ist auch hier am Außenumfang zumindest einer der Kontaktbuchseneinrichtungen zumindest eine eine Vertiefung bildende Ausformung vorgesehen, in welche bevorzugt zumindest ein Teil der Vielzahl der federnd gehaltenen Kontaktlamellen im gekoppelten Zustand rastbar ist. Eine Rastung gibt dem Anwender vorteilhaft eine taktile Rückmeldung über den betriebsfähig hergestellten Verbindungs- oder Kopplungszustand. Es kann somit vor einer Anwendung vermieden werden, dass das Werkzeug versehentlich nicht vollständig verbunden und/oder nicht wie vorgesehen bestromt ist. Die Gefahr von Anwendungsfehlern am Patienten wird verringert.

Bevorzugt weist weiter die Verbindungsvorrichtung zumindest ein Isolationselement mit einer ersten Ausnehmung an der ersten Position zur Aufnahme der ersten Kontaktlamelleneinrichtung und einer zweiten Ausnehmung an der zweiten Position zur Aufnahme der zweiten Kontaktlamelleneinrichtung auf; und sind Leiterverbindungen zum elektrischen Anschluss jeweils der ersten Kontaktlamelleneinrichtung und der zweiten Kontaktlamelleneinrichtung an Anschlüsse in dem Handstück durch das zumindest eine Isolationselement geführt. Um vorteilhaft gezielt einen Strom von dem chirurgischen Instrument auf zu operierende Körperteile übertragen zu können, kann wenigstens eines der Branchen zumindest abschnittsweise elektrisch leitend ausgebildet sein. Eine bei einer bipolaren Beaufschlagung einer Branche des chirurgischen Werkzeugs hierfür notwendige Stromführung kann somit vorteilhaft und transparent durch die Verbindungsvorrichtung geführt werden, ohne die Stromführung in den übrigen Komponenten zu beeinträchtigen.

Die Erfindung wird nachstehend mit weiteren Vorteilen und Wirkungen anhand bevorzugter Ausführungsbeispiele unter Bezugnahme auf die Zeichnung näher beschrieben. Es zeigen:
Fig. 1 eine schematische perspektivische Darstellung eines chirurgischen Instruments;
Fig. 2 eine vereinfachte Schnittansicht eines ersten Ausführungsbeispiels einer Verbindungsvorrichtung zur Verbindung eines Rohrschaftabschnitts mit einem Handstück des chirurgischen Instruments; und
Fig. 3 eine vereinfachte Schnittansicht eines zweiten Ausführungsbeispiels der Verbindungsvorrichtung zur Verbindung des Rohrschaftabschnitts mit dem Handstück des chirurgischen Instruments.

Gleiche oder funktional äquivalente Merkmale sind in den einzelnen Figuren mit denselben Bezugszeichen versehen.

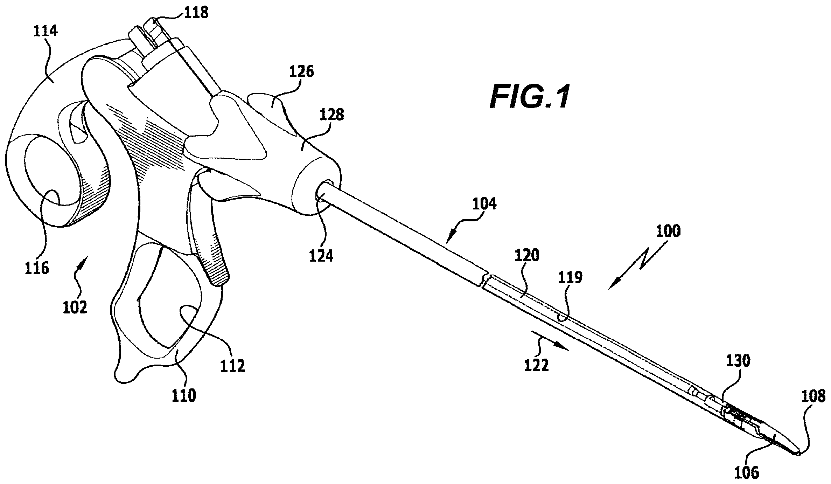

Ein beispielhaft in den Figuren 1 bis 3 dargestelltes chirurgisches Instrument 100 umfasst ein Handstück/Handgriff 102, einen rohrförmigen Werkzeugschaft 104 mit einer innenliegenden Kraftübertragungseinrichtung 120, eine erste Werkzeugbranche 106 und eine zweite Werkzeugbranche 108.

Das Handstück 102 hat ein feststehendes Griffstück 110 mit einer Fingeröffnung 112 und einen bewegbaren Betätigungshebel 114 mit einer Fingeröffnung 116. An dem Handgriff 102 kann beispielsweise ein Chirurg angreifen und durch eine Relativbewegung des bewegbaren Hebels 114 zu dem feststehenden Griffstück 110 vermittels der Kraftübertragungseinrichtung 120 die erste Branche 106 und/oder die zweite Branche 108 an einem Werkzeugkopf betätigen.

Ferner umfasst das Handstück 102 elektrische Anschlüsse 118 zur Kontaktierung und/oder zum Anlegen einer Spannung. Von den Anschlüssen 118 führen elektrische Verbindungen zu der ersten Branche 106 und/oder der zweiten Branche 108 und bestromen diese oder daran angeordnete Elektroden (nicht weiter dargestellt).

Der Werkzeug-/Rohrschaft 104 ist im Wesentlichen rotationssymmetrisch um eine Mittelachse ausgebildet und umfasst einen zylindrischen Hohlraum 119. Der Rohrschaft 104 ist somit als ein langgestreckter Schaft für den minimalinvasiven Einsatz des Instruments ausgebildet.

Das Handstück 102 ist mit dem Rohrschaft 104 und einer in dem zylindrischen Hohlraum 119 des Rohrschafts 104 geführten Zug-/Druckstange 120, welche die Kraftübertragungseinrichtung bildet, verbunden/gekoppelt, wobei eine Bewegung in einer Kraftübertragungsrichtung (Axialrichtung) 122 der Zug-/Druckstange 120 relativ zu dem Rohrschaft 104 und/oder eine Drehbewegung der Zug-/ Druckstange 120 relativ zu dem Rohrschaft 104 gewährleistet ist. Insbesondere kann hierbei die Zug-/Druckstange 120 drehfest oder drehbar gekoppelt an dem Handstück 102 angelenkt sein, und kann der Rohrschaft 104 um eine parallel zu der Kraftübertragungsrichtung 122 verlaufende Achse drehfest oder drehbar an dem Handstück 102 gelagert sein.

Zum Drehen des Rohrschafts 104 um die parallel zu der Kraftübertragungsrichtung 122 ausgerichtete Achse ist in dem in Fig. 1 gezeigten Beispiel an einem dem Handstück 102 zugewandten, proximalen Ende 124 des Werkzeugschafts 104 eine beispielsweise als Drehelement/Drehknopf 126 ausgebildete Betätigungseinrichtung 128 vorgesehen. Mittels der Betätigungseinrichtung 128 kann ein Chirurg, welcher das chirurgische Instrument 100 bedient, den Rohrschaft 104 besonders einfach um die parallel zu der Kraftübertragungsrichtung 122 ausgerichtete Achse (Schaftachse) drehen. An einem dem Handstück 102 abgewandten, distalen Ende 130 des Werkzeugschafts 104 sind die erste Branche 106 und die zweite Branche 108 relativ verschwenkbar zueinander angeordnet.

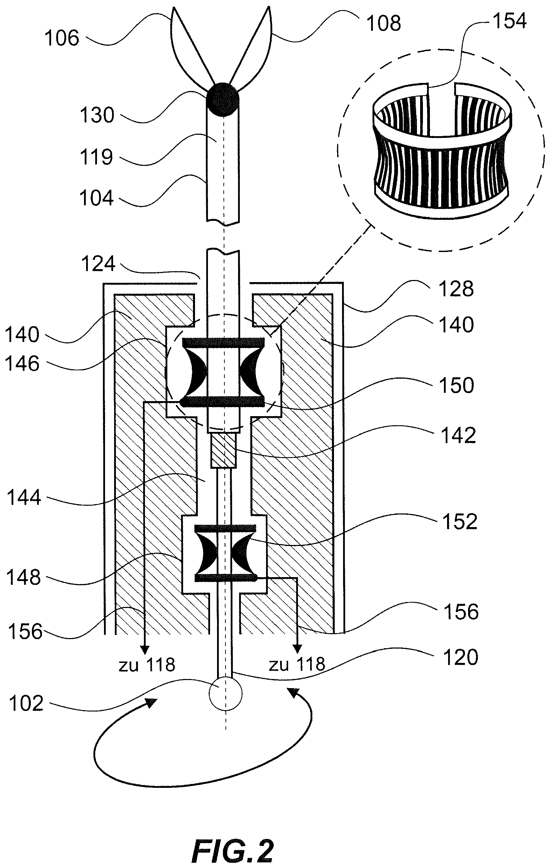

Während eine solche Betätigungseinrichtung 128 grundlegend auch erfindungsgemäß vorgesehen sein kann, um beispielsweise anwendungsbezogen eine manuelle Drehung des Rohrschafts 104 etwa zur Führung am Patienten oder zu dessen Ausrichtung und/oder Justierung durchführen zu können, beinhaltet erfindungsgemäß die Betätigungseinrichtung 128 ferner eine Verbindungsvorrichtung als Teil eines Stecksystems zur zumindest vorübergehenden Aufnahme eines zur einmaligen Verwendung bestimmten Werkzeugs (Single-Use-Werkzeug). Alternativ kann auch lediglich die Verbindungsvorrichtung ohne die Funktionalität der Betätigungseinrichtung 128 und/oder des Drehelements 126 vorhanden sein, beispielsweise wenn deren Betätigungs-Funktionen nicht benötigt oder konstruktiv bedingt nicht vorgesehen sein sollen. Der Einfachheit halber wird nachstehend und im Hinblick auf die vorgenannten Alternativen auch die Verbindungsvorrichtung mit dem Bezugszeichen 128 bezeichnet. Fig. 2 zeigt eine vereinfachte Schnittansicht eines ersten Ausführungsbeispiels der Verbindungsvorrichtung 128 zur Verbindung des Rohrschafts bzw. Rohrschaftabschnitts 104 mit dem Handstück 102 (in Fig. 2 nicht dargestellt bzw. auf ein kreisrundes Element reduziert) des chirurgischen Instruments. Im übertragenen Sinne entsprechen weiter in Fig. 2 die erste Branche 106, die zweite Branche 108, der zylindrische Hohlraum 119, das proximale Ende 124 und das distale Ende 130 den in Fig. 1 dargestellten Positionen.

Gemäß Fig. 2 sind innenseitig der im Handgriff eingehausten Verbindungsvorrichtung 128, die im Übrigen vorzugsweise rundförmig bzw. zylinderförmig und rotationssymmetrisch ausgestaltet ist, ohne dabei jedoch auf eine bestimmte äußere Formgebung beschränkt zu sein, Isolationselemente 140 und 142 vorgesehen, welche ein Aufnahmefutter des Handgriffs für das Werkzeug/den Werkzeugschaft bilden. Das Isolationselement 140 besteht vorzugsweise aus zumindest einem Formstück und vorteilhaft aus mehreren, beispielsweise zwei, Formstücken aus einem elektrisch isolierenden und zur Verwendung in der Medizintechnik geeigneten Material, beispielsweise einem Kunststoff, einem Harz, Gummi oder Kautschuk, oder einer Keramik, ohne darauf beschränkt zu sein. Beispielsweise kann eine Isolation auch mittels einer elektrisch isolierenden Beschichtung dargestellt werden.

Das Isolationselement 140 ist hinsichtlich seiner Größe an das Innenvolumen der Verbindungsvorrichtung 128 und den von dem Rohrschaft 104 benötigten Raum angepasst, d.h. sie füllt einerseits freies Volumen der Verbindungsvorrichtung 128 (distaler Abschnitt des Handgriffs) bis an deren Außenumfang (Handgriffgehäuse) im Wesentlichen aus, und weist außerdem zur Mittelachse des Rohrschafts 104 hin einen Aufnahmeschacht 144 und sich entlang dessen Längsrichtung und rundförmig konzentrisch um die Mittelachse des Rohrschafts 104 erstreckend zusätzlich zumindest eine erste Ausnehmung 146 und eine zweite Ausnehmung 148 zur Aufnahme jeweils einer ersten Kontaktlammelleneinrichtung 150 bzw. einer zweiten Kontaktlamelleneinrichtung 152 auf. Dabei sei darauf hingewiesen, dass das Isolationselement 140 auch einstückig (stoffeinstückig) mit dem Handgriff-Gehäuse ausgebildet sein kann.

Die Kontaktlamelleneinrichtungen (oder auch Einspannmanschetten) 150, 152 sind für geringen Übergangswiderstand und hohe Stromtragfähigkeit jeweils vorwiegend aus einem Metall hergestellt und beispielsweise mit Nickel, Silber oder Gold beschichtet, ohne jedoch darauf beschränkt zu sein, und können beispielsweise als ringförmiges Stanzteil in Blattfedertechnik oder als eine Flechtkorbstruktur konfiguriert sein. Zur besseren Veranschaulichung ist eine Kontaktlamelleneinrichtung 154 der genannten Art in Fig. 2 in einem Kreis aus durchbrochener Linie symbolhaft genauer gezeigt.

In dem ersten Ausführungsbeispiel der Verbindungsvorrichtung 128 kann die erste Kontaktlamelleneinrichtung 150 in einer beispielsweise käfigartigen, starren Federkorbkomponente (nicht gezeigt) aufgenommen sein, welche ortsfest in der ersten Ausnehmung 146 angeordnet ist. Der starre Federkorb gewährleistet, dass die erste Kontaktlamelleneinrichtung 150 im unbestückten Zustand der Verbindungsvorrichtung 128 weitestgehend frei von einwirkenden Kräften und insoweit unbelastet ist.

In einem mit dem Rohrschaft 104 bestückten Zustand der Verbindungsvorrichtung 128 werden die einzelnen Kontaktlamellen der ersten Kontaktlamelleneinrichtung 150 dadurch, dass der Außendurchmesser des Rohrschafts 104 größer ist als der kleinste Innendurchmesser der unbelasteten Kontaktlamelleneinrichtung 150, durch den Außendurchmesser des Rohrschafts 104 mit einer Druckkraft radial nach Außen beaufschlagt, welche die Kontaktlamellen gleichmäßig über den Umfang verteilt nach außen elastisch drückt. Als Reaktion darauf erzeugt die erste Kontaktlamelleneinrichtung 150 eine auf den Umfang des Rohrschafts 104 wirkende Haltekraft (Reibschluss). Diese Haltekraft, d.h. eine Spannwirkung der Kontaktlamelleneinrichtung 150 auf den Rohrschaft 104, ist so ausreichend groß ausgelegt, dass der gesamte Rohrschaft 104 während der Anwendung des chirurgischen Instruments in der Verbindungsvorrichtung 128 bzw. dem Handstück 102 axial fixiert wird, und so ausreichend klein gewählt, dass der Rohrschaft 104 nach der Anwendung des chirurgischen Instruments wieder aus der Verbindungsvorrichtung 128 bzw. dem Handstück 102 gezogen bzw. gelöst werden kann und ggf. auch im Handgriff rotiert werden kann.

Der Rohrschaft 104 kann zur Unterstützung der Haltekraft und der axialen Fixierung in der Verbindungsvorrichtung 128 an seinem Außenumfang mit einer zumindest stellenweise, alternativ ringförmig umlaufenden nutartigen Ausformung bzw. Vertiefung versehen sein, in welche Lamellen der ersten Kontaktlamelleneinrichtung 150 federnd rastend eingreifen können. Vorteilhaft wird hierdurch ein taktil und/oder hörbar erfassbares Einrasten an einer vorbestimmten Axial-Relativposition erzielt, so dass auch eine Rückmeldung hinsichtlich des Erreichens der vorbestimmten Axialposition des Rohrschafts 104 in der Verbindungsvorrichtung 128 erhalten werden kann.

In dem ersten Ausführungsbeispiel der Verbindungsvorrichtung 128 ist ferner die zweite Kontaktlamelleneinrichtung 152 als (axial wirkender) Schleifkontakt um die Zug-Druckstange 129 ausgebildet und als solcher ortsfest in der zweiten Ausnehmung 148 angeordnet. Die zweite Kontaktlamelleneinrichtung kann ebenfalls in einem käfigartigen, starren Federkorb (nicht gezeigt) aufgenommen sein, der gewährleistet, dass die zweite Kontaktlamelleneinrichtung 152 im unbestückten Zustand der Verbindungsvorrichtung 128 (kein Werkzeug in das Aufnahmefutter eingesteckt) weitestgehend frei von einwirkenden Kräften und insoweit unbelastet ist.

In dem mit dem Rohrschaft 104 bestückten Zustand der Verbindungsvorrichtung 128 werden die einzelnen Kontaktlamellen der zweiten Kontaktlamelleneinrichtung 152 dadurch, dass der Außendurchmesser der Zug-/Druckstange 120 größer ist als der kleinste Innendurchmesser der unbelasteten Kontaktlamelleneinrichtung, durch den Außendurchmesser der Zug-/Druckstange 120 mit einer Druckkraft radial beaufschlagt, welche die Kontaktlamellen gleichmäßig über den Umfang verteilt radial nach außen drückt. Als Reaktion darauf erzeugt die zweite Kontaktlamelleneinrichtung 152 eine auf den Umfang der Zug-/Druckstange 120 wirkende Einspannkraft (Reibschluss). Diese Einspannkraft, d.h. eine Spannwirkung der zweiten Kontaktlamelleneinrichtung 152 auf die Zug-/Druckstange 120, ist so klein bemessen, dass eine permanente Schleifkontaktierung zu der in Längsrichtung und ggf. rotatorisch beweglichen Zug-/Druckstange 120 geschaffen wird.

Zusammen mit der Erzeugung der Haltekraft durch die erste Kontaktlamelleneinrichtung 150 und der Erzeugung der Einspannkraft durch die zweite Kontaktlamelleneinrichtung 152 erfolgt durch die erste Kontaktlamelleneinrichtung 150 eine Bestromung des Rohrschafts 104 an dessen Außenumfang in der ersten Ausnehmung 146 und durch die zweite Kontaktlamelleneinrichtung 152 eine Bestromung der Zug-/Druckstange 120 an deren Außenumfang in der zweiten Ausnehmung 148. Zur Vermeidung eines Kurzschlusses sind der Rohrschaft 104 (Außenrohr) und die Zug-/Druckstange 120 durch das Isolationselement 142 radial gegeneinander isoliert. Zur Bestromung des bipolaren Rohrschaftinstruments sind im Weiteren die erste Kontaktlamelleneinrichtung 150 und die zweite Kontaktlamelleneinrichtung 152 jeweils mit leitenden Verbindungen 156 versehen, die von den Kontaktlamelleneinrichtungen 150, 152 weg und durch das Isolatorelement 140 verlaufend zu den Anschlüssen 118 am Handstück 102 geführt sind.

In anderen Worten wird das bipolare Rohrschaftinstrument bzw. dessen Werkzeug in der Verbindungsvorrichtung 128 bzw. dem Handstück 102 über zwei Kontaktlamelleneinrichtungen 150, 152 bestromt. Eine Kontaktlamelleneinrichtung 152 mit einer geringen Kraft ist an der Zug-/Druckstange 120 lokalisiert. Die andere Kontaktlamelleneinrichtung 150 mit einer großen Kraft ist am Außenrohr des Rohrschafts 104 lokalisiert. Die beiden Teile (Außenrohr und Zug-/Druckstange) sind gegeneinander isoliert. Die Kraft der anderen Kontaktlamelleneinrichtung 150 ist groß genug, um den gesamten Schaft 104 während der Anwendung im Handstück 102 axial zu fixieren. Sie ist außerdem klein genug, um den Schaft 104 wieder aus dem Handstück 102 lösen zu können. Die Zug-/Druckstange 120 wird über den Schleifkontakt der einen Kontaktlamelleneinrichtung 152 bestromt und kann am Schleifkontakt axial verschoben werden, ohne dass der elektrische Kontakt unterbrochen wird. Der Schaft 104 lässt sich im Handstück 102 vorzugsweise drehen.

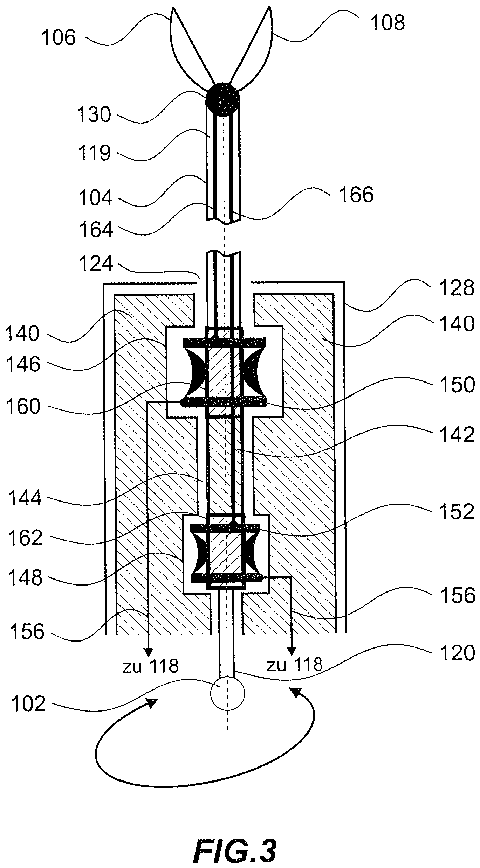

Fig. 3 zeigt eine vereinfachte Schnittansicht eines zweiten Ausführungsbeispiels der Verbindungsvorrichtung 128 zur Verbindung des Rohrschafts bzw. Rohrschaftabschnitts 104 mit dem Handstück 102 (in Fig. 3 nicht dargestellt bzw. auf ein kreisrundes Element reduziert) des chirurgischen Instruments. Im übertragenen Sinne entsprechen auch in Fig. 3 die erste Branche 106, die zweite Branche 108, der zylindrische Hohlraum 119, das proximale Ende 124 und das distale Ende 130 den in Fig. 1 dargestellten Positionen.

Gemäß Fig. 3 sind innenseitig der im Handgriff eingehausten Verbindungsvorrichtung 128, die im Übrigen vorzugsweise rundförmig bzw. zylinderförmig und rotationssymmetrisch ausgestaltet ist, ohne dabei jedoch auf eine bestimmte äußere Formgebung beschränkt zu sein, Isolationselemente 140 und 142 vorgesehen. Das Isolationselement 140 besteht vorzugsweise aus zumindest einem Formstück und vorteilhaft aus mehreren, beispielsweise zwei, Formstücken aus einem elektrisch isolierenden und zur Verwendung in der Medizintechnik geeigneten Material, beispielsweise einem Kunststoff, einem Harz, Gummi oder Kautschuk, oder einer Keramik, ohne darauf beschränkt zu sein.

Das Isolationselement 140 ist hinsichtlich seiner Größe an das Innenvolumen der Verbindungsvorrichtung 128 (entspricht dem distalen Abschnitt des Handgriffs) und den von dem Rohrschaft 104 benötigten Raum angepasst, d.h. sie füllt einerseits freies Volumen der Verbindungsvorrichtung 128 bis an deren Außenumfang (Griffgehäuse) im Wesentlichen aus, und weist außerdem zur Mittelachse des Rohrschafts 104 hin einen Aufnahmeschacht 144 und sich entlang dessen Längsrichtung und rundförmig konzentrisch um die Mittelachse des Rohrschafts 104 erstreckend zusätzlich zumindest eine erste Ausnehmung 146 und eine zweite Ausnehmung 148 zur Aufnahme jeweils einer ersten Kontaktlammelleneinrichtung 150 bzw. einer zweiten Kontaktlamelleneinrichtung 152 auf. Auch in diesem Fall kann die Verbindungseinrichtung 128 (stoff-) einstückig mit dem Griff ausgebildet sein.

Die erste und die zweite Kontaktlamelleneinrichtung 150, 152 sind für einen geringen Übergangswiderstand und hohe Stromtragfähigkeit vorwiegend aus einem Metall hergestellt und beispielsweise mit Nickel, Silber oder Gold beschichtet, ohne jedoch darauf beschränkt zu sein, und können beispielsweise als ringförmiges Stanzteil in Blattfedertechnik oder als ein Flechtkorb konfiguriert sein. Die in Fig. 2 zur besseren Veranschaulichung gezeigte Kontaktlamelleneinrichtung 154 der genannten Art ist in Fig. 3 weggelassen, obschon in Fig. 3 dieselbe grundlegende Struktur Verwendung finden kann.

In dem zweiten Ausführungsbeispiel der Verbindungsvorrichtung 128 ist die erste Kontaktlamelleneinrichtung 150 in einer beispielsweise käfigartigen, starren Federkorbkomponente (nicht gezeigt) aufgenommen, welche ortsfest in der ersten Ausnehmung 146 angeordnet ist, und ist die zweite Kontaktlamelleneinrichtung 152 in einer beispielsweise ebenfalls käfigartigen, starren Federkorbkomponente (nicht gezeigt) aufgenommen, welche ortsfest in der zweiten Ausnehmung 148 angeordnet ist. Die starren Federkörbe gewährleisten dabei, dass die erste und die zweite Kontaktlamelleneinrichtung 150, 152 im unbestückten Zustand der Verbindungsvorrichtung 128 weitestgehend frei von einwirkenden Kräften und insoweit unbelastet sind.

Ferner sind in dem zweiten Ausführungsbeispiel am Außenumfang des Rohrschafts 104 (nicht aber an der darin gelagerten Kraftübertragungsvorrichtung) eine erste Kontaktbuchseneinrichtung 160 und eine zweite Kontaktbuchseneinrichtung 162 in jeweiliger axialer Entsprechung zu den Ausnehmungen 146, 148, d.h. an einer ersten axialen Position der ersten Kontaktlamelleneinrichtung 150 und an einer zweiten axialen Position der zweiten Kontaktlamelleneinrichtung 152, und jeweils durch das Isolationselement 142 elektrisch von dem Rohrschaft 104 isoliert angeordnet. In einem mit dem Rohrschaft 104 bestückten Zustand der Verbindungsvorrichtung 128 werden die einzelnen Kontaktlamellen der ersten und der zweiten Kontaktlamelleneinrichtungen 150, 152 dadurch, dass der Außendurchmesser der Kontaktbuchseneinrichtungen 160, 162 größer ist als der kleinste Innendurchmesser der unbelasteten Kontaktlamelleneinrichtung, durch den Außendurchmesser der Kontaktbuchseneinrichtungen 160, 162 mit einer radialen Druckkraft beaufschlagt, welche die Kontaktlamellen gleichmäßig über den Umfang verteilt nach außen drückt. Als Reaktion darauf erzeugen die erste und die zweite Kontaktlamelleneinrichtung 150, 152 eine auf den Umfang der Kontaktbuchseneinrichtungen 160, 162 wirkende Haltekraft. Diese Haltekraft, d.h. eine Spannwirkung der Kontaktlamelleneinrichtungen 150, 152 auf die Kontaktbuchseneinrichtungen 160, 162 und damit auch auf den Rohrschaft 104, ist so ausreichend groß ausgelegt, dass der gesamte Rohrschaft 104 während der Anwendung des chirurgischen Instruments in der Verbindungsvorrichtung 128 bzw. dem Handstück 102 axial fixiert wird, und so ausreichend klein gewählt, dass der Rohrschaft 104 nach der Anwendung des chirurgischen Instruments wieder aus der Verbindungsvorrichtung 128 bzw. dem Handstück 102 gezogen bzw. gelöst werden kann und ggf. drehbar ist.

Der Rohrschaft 104 bzw. die Kontaktbuchseneinrichtungen 160, 162 kann bzw. können zur Unterstützung der Haltekraft und der axialen Fixierung in der Verbindungsvorrichtung 128 an dem jeweiligen Außenumfang mit einer zumindest stellenweise, alternativ ringförmig umlaufenden nutartigen Ausformung versehen sein, in welche Lamellen der ersten und/oder der zweiten Kontaktlamelleneinrichtung 150, 152 federnd rastend eingreifen können. Vorteilhaft wird hierdurch ein taktil und/oder hörbar erfassbares Einrasten an einer vorbestimmten Position erzielt, so dass auch eine Rückmeldung hinsichtlich des Erreichens einer vorbestimmten Position des Rohrschafts 104 in der Verbindungsvorrichtung 128 erhalten werden kann.

In dem zweiten Ausführungsbeispiel der Verbindungsvorrichtung 128 sind im Gegensatz zu dem ersten Ausführungsbeispiel beide Federkörbe bzw. Kontaktlamelleneinrichtungen 150, 152 zur Erzeugung einer Haltekraft vorgesehen, so dass beide Federkörbe bzw. Kontaktlamelleneinrichtungen 150, 152 gemeinsam das Werkzeug im Handstück 102 axial fixieren und kein Schleifkontakt (im Zusammenwirken mit der Kraftübertragungseinrichtung) ohne Beitrag zur Gesamthaltekraft benötigt wird.

Zusammen mit der Erzeugung der axialen Haltekraft durch die erste Kontaktlamelleneinrichtung 150 und der Erzeugung der axialen Haltekraft durch die zweite Kontaktlamelleneinrichtung 152 erfolgt durch die erste Kontaktlamelleneinrichtung 150 eine Bestromung des ersten Werkzeugs 106 über die am Umfang des Rohrschafts 104 angeordnete und gegenüber dem Rohrschaft 104 isolierte erste Kontaktbuchseneinrichtung oder (hülsenförmige) Buchse 160, die zur Stromführung mittels einer ersten Leiterverbindung 164, beispielsweise einer isolierend ummantelten Litze innerhall des Rohrschafts, mit der ersten Branche 106 verbunden ist, und erfolgt durch die zweite Kontaktlamelleneinrichtung 152 eine Bestromung der zweiten Branche 108 über die ebenfalls am Umfang des Rohrschafts 104 angeordnete und gegenüber dem Rohrschaft 104 isolierte zweite Kontaktbuchseneinrichtung oder (hülsenförmige) Buchse 162, die zur Stromführung mittels einer zweiten Leiterverbindung 166, beispielsweise einer isolierend ummantelten Litze innerhalb des Rohrschafts, mit der zweiten Branche 108 verbunden ist.

D.h., zur Vermeidung eines Kurzschlusses sind der Rohrschaft 104 (Außenrohr) und die erste und die zweite Kontaktbuchseneinrichtung oder Buchse 160, 162 gegeneinander isoliert, beispielsweise durch das Isolationselement 142, welches sich in diesem Fall zumindest von dem distalen Ende der ersten Kontaktbuchseneinrichtung 160 bis zu dem proximalen Ende der zweiten Kontaktbuchseneinrichtung 162 unter diesen hindurch auf dem Außenumfang des Rohrschafts 104 erstreckt. Zur Bestromung des bipolaren Rohrschaftinstruments sind im Weiteren die erste Kontaktlamelleneinrichtung 150 und die zweite Kontaktlamelleneinrichtung 152 jeweils mit leitenden Verbindungen 156 versehen, die von den Kontaktlamelleneinrichtungen 150, 152 weg und durch das Isolatorelement 140 verlaufend zu den Anschlüssen 118 am Handstück 102 geführt sind.

In anderen Worten wird das bipolare Rohrschaftinstrument in der Verbindungsvorrichtung 128 bzw. dem Handstück 102 über die zwei Kontaktlamelleneinrichtungen 150, 152, zwei Buchsen 160, 162 und zwei Leiter 164, 166, die an die zwei Buchsen 160, 162 angeschlossen sind und von den zwei Buchsen 160, 162 ausgehend durch den Rohrschaft 104 zu jeweils der ersten und der zweiten Branche 106, 108 führen, bestromt, so dass nicht die Zug-/Druckstange 120 und das Außenrohr, sondern die an die Buchsen angeschlossenen Litzen im Außenrohr den Strom leiten, wobei beide Federkörbe das Werkzeug gemeinsam in der Verbindungsvorrichtung 128 bzw. im Handstück 102 axial fixieren. Ein unter Umständen verschmutzungs- und verschleißanfälliger, und damit störanfälliger, Schleifkontakt an der Zug-/Druckstange 120 ist hier nicht erforderlich. Die Kraft der beiden Kontaktlamelleneinrichtungen 150, 152 ist groß genug, um den gesamten Schaft 104 während der Anwendung im Handstück 102 axial zu fixieren. Sie ist außerdem klein genug, um den Schaft 104 wieder aus dem Handstück 102 lösen zu können. Der Schaft 104 lässt sich im Handstück 102 drehen.

Vorzugsweise werden zwei identische Federkörbe und/oder Kontaktlamelleneinrichtungen 150, 152 mit dann in etwa hälftiger Haltekraftaufteilung verwendet. Es ist jedoch alternativ möglich, unterschiedliche Federkörbe bzw. Kontaktlamelleneinrichtungen 150, 152 mit dann unterschiedlicher Haltekraftaufteilung anzuordnen, um beispielsweise konstruktiven Besonderheiten und/oder Größenverhältnissen an der Verbindungseinrichtung 128 und/oder dem Handstück 102 entsprechen zu können.

Vorstehend wurde somit ein Stecksystem in Form einer Verbindungsvorrichtung 128 bzw. einer Koppelvorrichtung an einem Handstück 102 eines bipolaren chirurgischen Instruments zum Verbinden und Trennen eines bestromten, zur einmaligen Verwendung vorgesehenen Werkzeugschafts 104 und des wiederverwendbaren Handstücks 102 mittels Kontaktlamellen beschrieben. Die Verbindung ermöglicht dabei eine Drehbewegung, die Bestromung des Werkzeugs über einen festen Kontakt und einen Schleifkontakt, alternativ schleifkontaktlos über zwei feste Kontakte, sowie die axiale Fixierung des Werkzeugschafts 104 im Handstück 102 gegen ungewolltes Herausfallen.

Es versteht sich, dass bei den vorstehend beschriebenen Ausführungsbeispielen eines bipolaren chirurgischen Instruments weitere Isolationsstellen zur elektrischen Isolation strom- oder potenzialführender Teile oder Abschnitte an entsprechenden Stellen vorgesehen sein können. Es versteht sich ferner, dass den beschriebenen Ausführungsbeispielen lediglich beispielhafter Charakter zukommt und sich insoweit für den Fachmann Modifikationen ohne Weiteres ergeben können, ohne dass dadurch der durch die beigefügten Patentansprüche definierte Schutzumfang verlassen wird. Derartige Modifikationen können insbesondere die Art und die Funktionsweise der ersten und der zweiten Branchen 106, 108 und/oder die körperliche Ausgestaltung von Verbindungsvorrichtung 128 und/oder Handstück 102 betreffen. Ebenso unterliegen Komponenten wie Federkörbe, Kontaktlamellen(einrichtungen), Buchsen, Rohrschaft, Zug-/Druckstange, Isolation und dergleichen keinen besonderen Beschränkungen hinsichtlich Material und Formgebung, solange durch sie die erfindungsgemäße Wirkung und Funktionalität bereitgestellt und erzielt wird.

## Patentansprüche

1. Elektrochirurgisches Instrument der bipolaren und vorzugsweise Minimalinvasivbauart mit einem Handgriff und einem chirurgischen Werkzeug, wobei
der Handgriff (102) eine Strombeaufschlagungsvorrichtung (118, 156) aufweist für das wahlweise Beaufschlagen des chirurgischen Werkzeugs mit einem elektrischen Strom mittels eines vorzugsweise am Handgriff angeordneten Stromschalters und einem Aufnahmefutter (128), in welches das chirurgische Werkzeug an einem proximalen Ende eines Werkzeugschafts (104) mechanisch ein- und aussteckbar und dabei elektrisch an der Strombeaufschlagungsvorrichtung ein- und auskoppelbar ist, wobei das Aufnahmefutter (128) zwei in Einsteckrichtung des Werkzeugschafts (104) axial beabstandete, radial elastisch nachgebende sowie elektrisch voneinander isolierte Aufnahme-/Lagerungselemente (150, 152) zur radialen und/oder axialen Führung und/oder Sicherung des Werkzeugschafts (104) hat, welche mit der Strombeaufschlagungsvorrichtung (118, 156) elektrisch verbunden sind, um einen elektrischen Strom auf das eingesteckte chirurgische Werkzeug zu übertragen, und
das chirurgische Werkzeug an dem Werkzeugschaft (104) zwei axial beabstandete, elektrisch voneinander isolierte Schaftabschnitte aufweist, die dafür angepasst sind, mit den beiden Aufnahme-/Lagerungselementen (150, 152) im Aufnahmefutter (128) des Handgriffs (102) in mechanischen Führungs- und/oder Sicherungseingriff und gleichzeitig in elektrischen Kontakt zu kommen,
**dadurch gekennzeichnet, dass**
wenigstens eines der Aufnahme-/Lagerungselemente (150) einen radial einwärts gerichteten Vorsprung oder eine radial einwärts gerichtete Ausbauchung hat und der Vorsprung/die Ausbauchung dafür angepasst ist, mit einer radialen Einkerbung/Hinterschneidung am Werkzeugschaft (104) in Rasteingriff für ein axiales Sichern des Werkzeugschafts (104) im Aufnahmefutter (128) des Handgriffs (102) zu kommen.

2. Elektrochirurgisches Instrument nach Anspruch 1, **dadurch gekennzeichnet, dass** die Aufnahme-/Lagerungselemente (150, 152) jeweils eine radial elastisch aufweitbare Schaftmanschette vorzugsweise in Form eines Schleifrings, Kugellagers mit radial elastisch verschiebbaren Kugeln oder eines Feder-/Lamellenkorbs ausbilden.

3. Elektrochirurgisches Instrument nach Anspruch 1 oder 2, **dadurch gekennzeichnet, dass** die beiden Aufnahme-/Lagerungselemente (150, 152) dafür angepasst sind, zueinander unterschiedlich große Radialkräfte auf das eingesteckte Werkzeug auszuüben.

4. Elektrochirurgisches Instrument nach einem der vorangehenden Ansprüche, **dadurch gekennzeichnet, dass** das Werkzeug dafür angepasst ist, in den Handgriff (102) des chirurgischen Instruments eingesteckt zu werden, wobei der Werkzeugkopf am distalen Ende des Werkzeugschafts (104) mit Elektroden besetzt ist oder Elektroden aufweist, die dafür angepasst sind, in dem mechanischen Führungs- und/oder Sicherungseingriff und gleichzeitig elektrischen Kontakt mit der Strombeaufschlagungsvorrichtung (118, 156) verbunden zu werden.

5. Elektrochirurgisches Instrument nach einem der vorangehenden Ansprüche, **dadurch gekennzeichnet, dass** der Werkzeugschaft (104) zwei ineinander liegende Schaftelemente / Schaftstäbe / Schaftrohre (119, 120) hat, die relativ axial verschiebbar zueinander sind, und an zwei Werkzeugbranchen (106, 108) des Werkzeugkopfs angekoppelt sind, um eine mechanische Betätigungskraft auf diese für deren relative Bewegung zueinander zu übertragen.

6. Elektrochirurgisches Instrument nach Anspruch 5, **dadurch gekennzeichnet, dass** der eine Schaftabschnitt zur Stromübertragung an dem äußeren und der andere, Schaftabschnitt zur Stromübertragung an dem inneren Schaftelement/Schaftstab angeordnet ist, wobei die beiden Schaftelemente/Schaftstäbe elektrisch voneinander isoliert sind.

7. Elektrochirurgisches Instrument nach Anspruch 5, **dadurch gekennzeichnet, dass** nur an einem aus dem äußeren und dem inneren Schaftelement/Schaftstab beide Schaftabschnitte vorzugsweise in Form von elektrisch leitenden Büchsen oder Hülsen angeordnet sind, die elektrisch isoliert an dem einen Schaftelement/Schaftstab montiert und über elektrische Leitungen mit den Elektroden oder Branchen im Werkzeugkopf verbunden sind.

8. Elektrochirurgisches Instrument nach einem der vorangehenden Ansprüche, **dadurch gekennzeichnet, dass** das/die auf das äußere Schaftelement des Werkzeugschafts (102) wirkende(s) Aufnahme-/Lagerungselement(e) (150) als quasi fester Kontakt und das/die auf das innere Schaftelement wirkende(s) Aufnahme-/Lagerungselement(e) (152) als zumindest axial wirkender Schleifkontakt ausgebildet ist.

9. Elektrochirurgisches Instrument nach Anspruch 1, **dadurch gekennzeichnet, dass** der Handgriff als Mehrwegartikel und das Werkzeug als Einwegartikel konzipiert sind.

10. Chirurgisches Werkzeug mit einem Werkzeugschaft (104), der dafür ausgebildet ist, an seinem proximalen Ende mechanisch in ein Aufnahmefutter (128) eines elektrochirurgischen Instruments der bipolaren Bauart ein- und ausgesteckt und dabei elektrisch an einer Strombeaufschlagungsvorrichtung eines Handgriffs (102) des elektrochirurgischen Instruments ein- und ausgekoppelt zu werden, wofür am Werkzeugschaft (104) zwei axial beabstandete, elektrisch voneinander isolierte Schaftabschnitte vorgesehen sind, die dafür angepasst sind, mit zwei Aufnahme-/Lagerungselementen (150, 152) im Aufnahmefutter (128) des Handgriffs (102) in mechanischen Führungs- und/oder Sicherungseingriff und gleichzeitig in elektrischen Kontakt zu kommen, **dadurch gekennzeichnet dass** zumindest eine der beiden axial beabstandeten, elektrisch voneinander isolierten Schaftabschnitte am Werkzeugschaft (104) mit einer radialen Einkerbung/Hinterschneidung ausgebildet ist, die dafür ausgebildet ist, um in Rasteingriff mit dem zugehörigen Aufnahme-/Lagerungselement (150, 152) für ein axiales Sichern des Werkzeugschafts (104) im Aufnahmefutter (128) des Handgriffs (102) zu kommen.

11. Chirurgisches Werkzeug nach Anspruch 10, **dadurch gekennzeichnet, dass** der Werkzeugschaft (104) zwei ineinander liegende Schaftelemente/Schaftstäbe/Schaftrohre (119, 120) hat, die relativ axial verschiebbar zueinander sind, und an zwei Werkzeugbranchen (106, 108)) eines distal am Werkzeugschaft (104) angeordneten Werkzeugkopfs angekoppelt sind, um eine mechanische Betätigungskraft auf diese für deren relative Bewegung zueinander zu übertragen.

12. Chirurgisches Werkzeug nach Anspruch 11, **dadurch gekennzeichnet, dass** der eine Schaftabschnitt zur Stromübertragung an dem äußeren und der andere Schaftabschnitt zur Stromübertragung an dem inneren Schaftelement/Schaftstab angeordnet ist, wobei die beiden Schaftelemente/Schaftstäbe elektrisch voneinander isoliert sind.

13. Chirurgisches Werkzeug nach Anspruch 11, **dadurch gekennzeichnet, dass** nur an einem aus dem äußeren und dem inneren Schaftelement/Schaftstab beide Schaftabschnitte vorzugsweise in Form von elektrisch leitenden Büchsen oder Hülsen angeordnet sind, die elektrisch isoliert an dem einen Schaftelement/Schaftstab montiert und über elektrische Leitungen mit den Elektroden oder Branchen im Werkzeugkopf verbunden sind.

## Claims

1. Electrosurgical instrument of a bipolar and preferably minimally invasive type with a handle and a surgical tool, wherein
the handle (102) comprises a current supply device (118, 156) for selectively supplying an electrical current to the surgical tool by means of a power switch preferably located on the handle and a receiving chuck (128) into which the surgical tool is mechanically attached to engage and disengage at a proximal end of a tool shaft (104) and can thereby electrically be connected and disconnected to the current supply device, wherein the receiving chuck (128) has two in the insertion direction of the tool shaft (104) axially spaced, radially elastically yielding and electrically isolated from each other receiving/storage elements (150, 152) for radially and/or axially guiding and/or securing the tool shaft (104), which are electrically connected to the current supply device (118, 156), for transmitting an electric current to the inserted surgical tool, and
the surgical tool on the tool shaft (104) has two axially spaced, electrically isolated shaft portions which are adapted to be in mechanical guiding and/or securing engagement with the two receiving/storage elements (150, 152) in the receiving chuck (128) of the handle (102) and at the same time come into electrical contact
**characterized in that**
at least one of the receiving/storage elements (150) has a radially inwardly directed projection or a radially inwardly directed bulge, and the projection/bulge is adapted for snap engagement with a radial notch/undercut on the tool shaft (104) for axially securing the tool shaft (104) in the receiving chuck (128) of the handle (102).

2. Electrosurgical instrument according to claim 1, **characterized in that** the receiving/storage elements (150, 152) each form a radially elastically expandable stem sleeve, preferably in the form of a slip ring, ball bearing with radially elastically displaceable balls or a spring/lamella cage.

3. Electrosurgical instrument according to claim 1 or 2, **characterized in that** the two receiving/storage elements (150, 152) are adapted to exert mutually different radial forces on the inserted tool.

4. An electrosurgical instrument according to any one of the preceding claims, **characterized in that** the tool is adapted to be inserted into the handle (102) of the surgical instrument, wherein the tool head is fitted with electrodes or having electrodes at the distal end of the tool shaft (104), which are adapted to be connected to the mechanical guiding and/or securing engagement, and at the same time having electrical contact with the current supply device (118, 156).

5. Electrosurgical instrument according to one of the preceding claims, **characterized in that** the tool shaft (104) has two mutually nested shaft elements / shaft rods / shaft tubes (119, 120) which are relatively axially displaceable relative to each other, and two tool branches (106, 108) of the tool head are coupled, and for transmitting a mechanical actuating force to these for their relative movement to each other.

6. Electrosurgical instrument according to claim 5, **characterized in that** the one shaft portion is arranged for power transmission to the outer, and the other shaft portion is arranged for power transmission to the inner shaft element / shaft rod, wherein the two shaft elements / shaft rods are electrically isolated from each other.

7. Electrosurgical instrument according to claim 5, **characterized in that** only at one of the outer and the inner shaft element / shaft rod both shaft portions are preferably arranged in the form of electrically conductive bushes or sleeves which are mounted electrically isolated on the one shaft element / shaft rod, and are connected via electrical lines to the electrodes or branches in the tool head.

8. Electrosurgical instrument according to one of the preceding claims, **characterized in that** the on the outer shaft member of the tool shaft (102) acting receiving/storage element(s) (150) are arranged as a quasi-solid contact, and the on the inner shaft member acting receiving/storage element(s) (152) is/are designed as at an at least axially acting sliding contact.

9. Electrosurgical instrument according to claim 10, **characterized in that** the handle is designed as a reusable item and the tool as a disposable item.

10. A surgical tool having a tool shaft (104) adapted to mechanically engage and disengage at its proximal end in a receiving chuck (128) of a bipolar type electrosurgical instrument and thereby electrically engaging and disengaging with a current supply device of a handle (102) of the electrosurgical instrument, for which on the tool shaft (104) two axially spaced, electrically insulated shaft portions are arranged, which are adapted with two receiving/storage elements (150,152) in the receiving chuck (128) of the handle (102) in mechanical guiding and/or securing engagement, and at the same time come into electrical contact, **characterized in that** at least one of the two axially spaced, electrically insulated shaft portions on the tool shaft (104) is formed with a radial notch / undercut, which is adapted for a snap engagement with the associated receiving/storage element (150, 152) for axially securing the tool shaft (104) in the receiving chuck (128) of the handle (102).

11. A surgical tool according to claim 10, **characterized in that** the tool shaft (104) has two nested shaft elements / shaft rods / shaft tubes (119, 120) which are relatively axially displaceable relative to each other, and are coupled to two tool branches (106, 108) of a distally arranged tool head on a tool shaft (104) for transmitting a mechanical actuating force on the tool head for their relative movement to each other.

12. A surgical tool according to claim 10, **characterized in that** the one shaft portion is arranged for power transmission to the outer and the other shaft portion for power transmission to the inner shaft element / shaft rod, wherein the two shaft elements / shaft rods are electrically isolated from each other.

13. Surgical tool according to claim 11, **characterized in that** only at one of the outer and the inner shaft element / shaft rod both shaft portions are preferably arranged in the form of electrically conductive bushes or sleeves, which are mounted electrically isolated on the one shaft element / shaft rod and via electrical lines connected to the electrodes or branches in the tool head.

## Revendications

1. Instrument électrochirurgical du type bipolaire et de préférence invasif au minimum avec une poignée et un outil chirurgical, dans lequel :
la poignée (102) présente un dispositif d'alimentation en courant (118, 156) pour l'alimentation au choix de l'outil chirurgical par un courant électrique au moyen d'un commutateur agencé de préférence sur la poignée et une gaine réceptrice (128), dans laquelle l'outil chirurgical peut être enfiché ou dégagé mécaniquement sur une extrémité proximale d'une queue d'outil (104) et peut être en l'occurrence couplé et désaccouplé électriquement sur le dispositif d'alimentation en courant, dans lequel la gaine réceptrice (128) a deux éléments de réception/logement (150, 152) espacés axialement dans la direction d'enfichage de la queue d'outil (104), élastiques radialement ainsi qu'isolés l'un de l'autre électriquement pour guider et/ou bloquer la queue d'outil (104) radialement et/ou axialement, qui sont connectés électriquement au dispositif d'alimentation en courant (118, 156) afin de transmettre un courant électrique à l'outil chirurgical enfiché, et
l'outil chirurgical présente sur la queue d'outil (104) deux sections de queue espacées axialement et isolées l'une de l'autre électriquement, qui sont adaptées pour venir en prise mécanique de guidage et/ou de blocage et simultanément en contact électrique avec les deux éléments de réception/logement (150, 152) dans la gaine réceptrice (128) de la poignée (102),
**caractérisé en ce que :**
au moins l'un des éléments de réception/logement (150) a une saillie dirigée radialement vers l'intérieur ou un bossage dirigé radialement vers l'intérieur et la saillie ou le bossage est adapté(e) de manière à venir en prise d'encliquetage avec une rainure/contre-dépouille radiale sur l'arbre d'outil (104) pour assurer un blocage axial de la queue d'outil (104) dans la gaine réceptrice (128) de la poignée (102).

2. Instrument électrochirurgical selon la revendication 1, **caractérisé en ce que** les éléments de réception/logement (150, 152) forment respectivement une manchette de queue qui peut s'évaser radialement de manière élastique sous la forme d'une bague collectrice, d'un roulement à billes avec des billes qui coulissent radialement de manière élastique ou d'une cage à ressort/lamelles.

3. Instrument électrochirurgical selon la revendication 1 ou 2, **caractérisé en ce que** les deux éléments de réception/logement (150, 152) sont adaptés de sorte à exercer des forces radiales de grandeur mutuelle différente sur l'outil enfiché.

4. Instrument électrochirurgical selon l'une quelconque des revendications précédentes, **caractérisé en ce que** l'outil est adapté en sorte d'être enfiché dans la poignée (102) de l'instrument chirurgical, dans lequel la tête de l'outil est dotée d'électrodes ou présente des électrodes à l'extrémité distale de la queue d'outil (104), lesquelles électrodes sont adaptées pour être connectées dans la prise mécanique de guidage et/ou de blocage et simultanément en contact électrique avec le dispositif d'alimentation en courant (118, 156).

5. Instrument électrochirurgical selon l'une quelconque des revendications précédentes, **caractérisé en ce que** la queue d'outil (104) a deux éléments de queue / tiges de queue / tubes de queue (119, 120) se situant l'un(e) à l'intérieur de l'autre, qui peuvent être déplacés l'un(e) par rapport à l'autre axialement et sont couplés à deux branches d'outil (106, 108) de la tête d'outil pour transmettre une force de commande mécanique à celles-ci pour leur déplacement relatif mutuel.

6. Instrument électrochirurgical selon la revendication 5, **caractérisé en ce que** la première section de queue est agencée pour transmettre du courant à l'élément de queue ou à la tige de queue externe et l'autre section de queue pour transmettre du courant à l'élément de queue ou à la tige de queue interne, dans lequel les deux éléments de queue ou tiges de queue sont isolés électriquement l'un(e) de l'autre.

7. Instrument électrochirurgical selon a revendication 5, **caractérisé en ce que,** uniquement sur l'un(e) de l'élément de queue / de la tige de queue externe et interne, les deux sections de queue sont agencées de préférence sous la forme de bagues ou de douilles électroconductrices, qui sont montées isolées électriquement sur le premier élément de queue / la première tige de queue et sont connectées par des lignes électriques aux électrodes ou aux branches de la tête d'outil.

8. Instrument électrochirurgical selon l'une quelconque des revendications précédentes, **caractérisé en ce que** le ou les éléments de réception/logement (150) agissant sur l'élément de queue externe de la queue d'outil (102) est ou sont conçus comme un contact quasi fixe et le ou les éléments de réception/logement (152) agissant sur l'élément de queue interne sont conçus comme un contact glissant agissant au moins axialement.

9. Instrument électrochirurgical selon la revendication 1, **caractérisé en ce que** la poignée est conçue comme un article à voies multiples et l'outil est conçu comme un article à voie unique.

10. Outil chirurgical comprenant une queue d'outil (104) qui est conçue pour être enfichée et dégagée mécaniquement dans une gaine réceptrice (128) d'un instrument électrochirurgical du type bipolaire à son extrémité proximale et, en l'occurrence, pour être couplée et désaccouplée électriquement sur un dispositif d'alimentation en courant d'une poignée (102) de l'instrument électrochirurgical, moyennant quoi il est prévu sur la queue d'outil (104) deux sections de queue espacées axialement et isolées électriquement l'une de l'autre, qui sont adaptées pour venir en prise de guidage et/ou de blocage mécanique et simultanément en contact électrique avec deux éléments de réception/logement (150, 152) dans la gaine réceptrice (128) de la poignée (102), **caractérisé en ce qu'**au moins l'une des deux sections de queue espacées axialement et isolées électriquement l'une de l'autre est conçue sur la queue d'outil (104) avec une rainure/contre-dépouille radiale qui est conçue pour venir en prise d'encliquetage avec l'élément de réception/logement associé (150, 152) pour assurer un blocage axial de la queue d'outil (104) dans la gaine réceptrice (128) de la poignée (102).

11. Outil chirurgical selon la revendication 10, **caractérisé en ce que** la queue d'outil (104) a deux éléments de queue ou tiges de queue ou tubes de queue (119, 120) disposés l'un(e) dans l'autre, qui peuvent être déplacés axialement l'un(e) par rapport à l'autre et sont couplés sur deux branches d'outil (106, 108) d'une tête d'outil agencée de manière distale sur la queue d'outil (104) pour transmettre une force de commande mécanique sur celles-ci pour leur déplacement relatif mutuel.

12. Outil chirurgical selon la revendication 11, **caractérisé en ce que** la première section de queue est agencée pour transmettre du courant à l'élément de queue ou à la tige de queue externe et l'autre section de queue pour transmettre du courant à l'élément de queue / à la tige de queue interne, dans lequel les deux éléments de queue / tiges de queue sont isolés électriquement l'un(e) de l'autre.

13. Outil chirurgical selon la revendication 11, **caractérisé en ce que**, uniquement sur l'un(e) de l'élément de queue ou de la tige de queue externe et de l'élément de queue ou de la tige de queue interne, les deux sections de queue sont agencées de préférence sous la forme de bagues ou de douilles électroconductrices, qui sont montées de manière isolée électriquement sur le premier élément de queue ou la première tige de queue et sont connectées via des lignes électriques aux électrodes ou aux branches de la tête d'outil.
